# EUROPÄISCHE PATENTANMELDUNG

(11) **EP 4 205 724 A1**
(43) Veröffentlichungstag der Anmeldung: **05.07.2023**
(21) Anmeldenummer: 21218194.5
(22) Anmeldetag: 29.12.2021
(51) Int. Cl.: A61K 6/77, A61K 6/887, A61K 6/889, A61K 6/30, A61K 6/61, A61K 6/62, C03C 3/062, C03C 3/091, C03C 4/00, C03C 12/00, C03C 15/00, C03C 17/30, C03C 23/00

(54) **SELBSTHAFTENDE DENTALE KOMPOSITZEMENTE MIT GUTER TRANSPARENZ AUF BASIS SÄUREBEHANDELTER FÜLLSTOFFE**

(71) Anmelder: Ivoclar Vivadent AG, 9494 Schaan (LI)
(72) Erfinder: MOSZNER, Norbert, 9495 Triesen (LI); CATEL, Yohann, 9475 Rans (Sevelen) (CH); GIANASMIDIS, Alexandros, 9436 Balgach (CH); CATEL, Delphine, 9475 Rans (Sevelen) (CH); GRABENBAUER, Barbara, 9445 Rebstein (CH); RITZBERGER, Christian, 9472 Grabs (CH); DITTMER, Marc, 6800 Feldkirch (AT); BIELEC, Philipp, 88142 Wasserburg (DE); BROT, Andy, 9496 Balzers (LI); HALDNER, Tim, 9493 Mauren (LI)
(74) Vertreter: Uexküll & Stolberg

(57) **Zusammenfassung**

Radikalisch polymerisierbarer Dentalwerkstoff, der mindestens ein radikalisch polymerisierbares Monomer ohne Säuregruppe, mindestens ein säuregruppenhaltiges radikalisch polymerisierbares Monomer, mindestens einen Fluoroaluminiumsilikatglasfüllstoff und/oder röntgenopaken Glasfüllstoff und mindestens einen Initiator für die radikalische Polymerisation enthält, wobei der Füllstoff ein säurebehandelter Fluoroaluminiumsilikatglasfüllstoff und/oder Glasfüllstoff ist.

## Beschreibung

Die vorliegende Erfindung betrifft radikalisch polymerisierbare, selbsthaftende Komposite mit verbesserter Transparenz, die sich insbesondere als Dentalwerkstoffe eignen, z.B. als dentale Zemente, Füllungskomposite oder Verblendmaterialen sowie zur Herstellung von Inlays, Onlays oder Kronen.

Komposite werden im Dentalbereich hauptsächlich zur Herstellung von direkten und indirekten Füllungen, d.h. als direkte und indirekte Füllungskomposite, sowie als Zemente eingesetzt. Die polymerisierbare organische Matrix der Komposite besteht meistens aus einer Mischung von Monomeren, Initiator-Komponenten und Stabilisatoren. Als Monomere werden gewöhnlich Mischungen von Dimethacrylaten eingesetzt, die auch monofunktionelle und funktionalisierte Monomere enthalten können. Häufig eingesetzte Dimethacrylate sind 2,2-Bis[4-(2-hydroxy-3-methacryloyloxy-propyl)phenyl]propan (Bis-GMA), 1,6-Bis-[2-methacryloyloxyethoxycarbonylamino]-2,2,4-trimethylhexan (UDMA), die eine hohe Viskosität haben und Polymerisate mit guten mechanischen Eigenschaften und geringem Polymerisationsschrumpf ergeben. Als reaktive Verdünner finden vor allem Triethylenglycoldimethacrylat (TEGDMA), 1,10-Decandioldimethacrylat (D₃MA) oder Bis(3-methacryloyloxy-methyl)tricyclo-[5.2.1.0^{2,6}]decan (DCP) Anwendung. Monofunktionelle Methacrylate, wie z.B. p-Cumylphenoxyethylenglycolmethacrylat (CMP-1E), eignen sich ebenfalls zur Reduzierung der Viskosität und bewirken darüber hinaus eine Verringerung der Netzwerkdichte und einen erhöhten Doppelbindungsumsatz.

Zur Herstellung selbsthaftender Komposite werden stark saure Haftmonomere eingesetzt, wie z.B. 10-Methacryloyloxydecyldihydrogenphosphat (MDP), das die Zahnhartsubstanz ätzt und durch Ionenbeziehung eine Haftung auf Schmelz/Dentin bewirkt. Haftmonomere verleihen Kompositen selbsthaftende Eigenschaften und ermöglichen damit den Einsatz der Komposite ohne eine Vorbehandlung der Zahnhartsubstanz mit einem Schmelz-Dentin-Adhäsiv, was deren Verwendung besonders attraktiv macht.

Neben der organischen Matrix enthalten Komposite einen oder mehrere Füllstoffe, die meist mit einem polymerisationsfähigen Haftvermittler, wie z.B. 3-Methacryloyloxypropyltrimethoxysilan, oberflächenmodifiziert sind. Füllstoffe verbessern die mechanischen Eigenschaften (Festigkeit, Elastizitätsmodul, Abrasionsfestigkeit) und die Verarbeitungseigenschaften (Pastenkonsistenz, Stopfbarkeit) der Werkstoffe und verleihen ihnen Röntgenopazität.

Problematisch ist, dass saure Haftmonomere mit Füllstoffen häufig nachteilige Wechselwirkungen eingehen. Beispielsweise werden die sauren Haftmonomere durch die Bildung unlöslicher Salze an die Oberfläche der Füllstoffe gebunden, oder sie bilden bei der Lagerung mit aus den Füllstoffen herausgelösten Ionen schwerlösliche Salze. Dies führt zu einer deutlichen Verringerung der Haftmonomerkonzentration in der Harzmatrix, was mit einer Verminderung oder sogar einem Verlust der Hafteigenschaften verbunden ist. Komposite mit sauren Haftmonomeren haben daher nur eine begrenzte Lagerstabilität.

Die Aushärtung methacrylatbasierender Dentalmaterialien erfolgt durch radikalische Polymerisation, wobei je nach Anwendungsgebiet radikalische Photoinitiatoren, thermische Initiatoren oder Redox-Initiatorsysteme eingesetzt werden. Dualhärtende Systeme enthalten eine Kombination von Photoinitiatoren und Redoxinitiatoren.

Kompositzemente enthalten in der Regel Redoxsysteme, weil diese auch dann eine ausreichende Härtung gewährleisten, wenn eine Lichthärtung wegen einer unzureichenden Durchstrahlung nicht möglich ist. Meist werden Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) basieren. Da die Radikalbildung bei DBPO/Amin-basierenden Redoxinitiatorsystemen durch starke Säuren und damit auch durch stark saure Haftmonomere sehr beeinträchtigt wird, werden bevorzugt Cumolhydroperoxid-haltige Redoxinitiatorsysteme in Kombination mit Thioharnstoffen, wie z.B. Acetylthioharnstoff, eingesetzt.

Um eine ausreichende Lagerstabilität der Redoxinitiatoren zu gewährleisten, werden Redox-Initiatorsystem-basierende Materialien meist als sog. 2-Komponenten-Systeme (2K) eingesetzt, wobei das Oxydationsmittel (Peroxid- oder Hydroperoxid) und das Reduktionsmittel (Amine, Sulfinsäuren, Barbiturate, Thioharnstoffe etc.) in voneinander getrennte Komponenten eingearbeitet werden. Diese werden erst kurz vor der Anwendung miteinander gemischt. Zum Mischen werden zunehmend Doppelschubspritzen verwendet, die getrennte zylindrische Kammern zur Aufnahme der Komponenten aufweisen. Die Komponenten werden mit zwei miteinander verbundenen Kolben gleichzeitig aus den Kammern herausgedrückt und in einer Düse miteinander gemischt. Um möglichst homogene Mischungen zu erhalten, ist es vorteilhaft, die Komponenten in etwa gleich großen Volumenanteilen miteinander zu mischen.

Herkömmliche Befestigungszemente, wie z.B. ZnO-Eugenol-Zemente, Zinkphosphat-Zemente, Glasionomer-Zemente (GIZ) und harzmodifizierte Glasionomer-Zemente (Resin Modified Glass Ionomer Cements: RMGI) eigenen sich nicht zur Anwendung mit Doppelschubspritzen, weil sie eine Pulverkomponente enthalten, was ein Mischen der Komponenten erheblich erschwert. Außerdem haben Glasionomerzemente nur eine geringe Transparenz und relativ schlechte mechanische Eigenschaften.

Konventionelle Glasionomer-Zemente (GIZ) enthalten als flüssige Komponente eine wässrige Lösung einer hochmolekularen Polyacrylsäure (PAA, zahlenmittlere Molmasse größer 30 000 g/mol) oder eines Copolymers vergleichbarer Molmasse aus Acrylsäure und Itaconsäure und als Pulverkomponente ein Calcium-Fluor-Aluminium-Glas. Sie härten nach dem Mischen der Komponenten durch eine rein ionische lonomerbildung aus. Nachteilig an Glasionomerzementen sind ihre geringe Transparenz und ihre schlechten mechanischen Eigenschaften.

Harzmodifizierte Glasionomer-Zemente (Resin Modified Glass Ionomer Cements: RMGI) enthalten zusätzlich hydrophile Monomere, wie z.B. 2-Hydroxyethylmethacrylat (HEMA). Ihre Aushärtung erfolgt sowohl durch eine Säure-Base-Reaktion als auch durch radikalische Polymerisation. Sie zeichnen sich gegenüber herkömmlichen GIZ durch eine verbesserte Biegefestigkeit aus.

Die US 8,053,490 B2 offenbart Fluorid freisetzende Dentalwerkstoffe, die Fluoroaluminiumsilikatglasfüllstoff (FAS) enthalten, der mit einer wässrigen Lösung eines säuregruppenhaltigen Monomers oder Oligomers umgesetzt wurde. Das säuregruppenhaltige Monomer oder Oligomer wird dabei an die Füllstoffoberfläche gebunden und soll eine Reaktion des Füllstoffs mit reaktiven Bestandteilen des Zements verhindern. Die Werkstoffe zeichnen sich durch eine geringe Transparenz und unbefriedigende mechanische Eigenschaften aus.

Die JP 20116030741 A1 offenbart Dentalwerkstoffe, die als Füllstoff ein Fluoroboroaluminiumsilikatglas enthalten, das mit einem Silan und einer polymeren Carbonsäure oberflächenmodifiziert ist. Die Füllstoffe weisen eine sphärische Struktur auf und sollen eine dauerhafte Freisetzung von Fluoridionen und anderen Ionen aufweisen. Die Dentalwerkstoffe zeigen keine Selbsthaftung auf Dentin und Schmelz und haben nur unbefriedigende mechanische Eigenschaften.

Die US 2016/0324729 A1 offenbart Füllstoffe für Glasionomerzemente, deren Oberfläche zunächst silanisiert und dann mit einer ungesättigten Carbonsäure modifiziert wird, die über ein Siliciumatom an die Füllstoffoberfläche gebunden wird. Die Carboxygruppe der Carbonsäure soll mit den Bestandteilen des Glasionomerzements wechselwirken und den Zement auf diese Weise stabilisieren. Die Werkstoffe haben nur eine mäßige Transparenz und schlechte mechanische Eigenschaften.

Die CN 102976618 A offenbart kostengünstige Glasfüllstoffe für wasserbasierende Glasionomerzemente, die 25-35 Gew.-% Al₂O₃, 30-45 Gew.-% SiO₂, 2-8 Gew.-% Na₂O, 5-15 Gew.-% CaF₂ und 5-8 Gew.-% SrO und/oder CaO enthalten. Die Glaspulver werden bei Raumtemperatur mit Salzsäure oder Essigsäure behandelt und anschließend getrocknet. Sie sollen eine stabile Qualität und gute mechanische Eigenschaften haben und kontinuierlich Fluoridionen freisetzen. Die Glasionomerzemente haben nur eine geringe Transparenz und schlechte mechanische Eigenschaften.

Dai et al., Int. J. Nanomedicine 14 (2019) 9185, haben gezeigt, dass die Oberflächenbehandlung von basischen ZrO₂-Füllstoffen mit 10-Methacryloyloxydecyldihydrogenphosphat (MDP) die Biegefestigkeit von dentalen Kompositen verbessern kann und dass eine vorherige Beschichtung der ZrO₂-Partikel mit Zr(OH)₄ die Bindung von MDP an das Zirkonoxid verbessert. Dentalwerkstoffe auf der Basis der oberflächenbehandelten Füllstoffe sind nicht selbsthaftend und haben nur eine geringe Transparenz.

Gemäß US 8,071,662 B2 soll die Oberflächenmodifizierung von basischen Füllstoffen, wie z.B. ZrO₂, mit einer starken Säure, wie z.B. 3-Methacryloyloxypropylsulfonsäure, die Lagerstabilität von selbstätzenden Dentalwerkstoffen verbessern. Die offenbarten Dentalwerkstoffe sind nicht selbsthaftend und haben eine unbefriedigende Transparenz.

DE 24 46 546 A1 offenbart die Behandlung von natürlich vorkommendem Siliciumdioxid mit einer wasserhaltigen Mineralsäure zum Entfernen säurelöslicher Verunreinigungen. Das Siliciumdioxid soll sich als Füllstoff für dentale Zwecke eignen. Röntgenopake oder selbsthaftende Dentalwerkstoffe werden nicht offenbart.

Die US 2001/0034309 A1 offenbart die Behandlung anorganischer Füllstoffe mit Persäuren oder Persäuresalzen zum Entfernen organischer Substanzen, z.B. von Kohlenstoff. Durch das Entfernen organischer Stoffe soll die Bindung von Silanhaftvermittlern an die Füllstoffoberfläche und damit die Einbindung der Füllstoffe in die organische Matrix von radikalisch polymerisierbaren Dentalwerkstoffen verbessert werden. Selbsthaftende Materialien werden nicht offenbart.

Der Erfindung liegt die Aufgabe zugrunde, lagerstabile, selbsthaftende dentale Komposite mit guter Transparenz und guten mechanischen Eigenschaften bereitzustellen, die sich als 2-Komponentensysteme gut mit Doppelschubspritzen mischen und applizieren lassen. Die Komposite sollen sich insbesondere als dentale Befestigungszemente eignen und röntgenopak sein.

Diese Aufgabe wird durch füllstoffhaltige, radikalisch polymerisierbare Zusammensetzungen gelöst, die mindestens ein radikalisch polymerisierbares Monomer, mindestens ein saures, radikalisch polymerisierbares Monomer, mindestens einen Fluoroaluminiumsilikatglasfüllstoff und/oder röntgenopaken Glasfüllstoff und mindestens einen Initiator für die radikalische Polymerisation enthalten. Die Zusammensetzungen sind dadurch gekennzeichnet, dass der Füllstoff mit Säure vorbehandelt wurde. Es wurde überraschend gefunden, dass die Säurebehandlung des Füllstoffs eine deutliche Erhöhung der Lagerstabilität der Zusammensetzungen bewirkt.

Die erfindungsgemäßen Zusammensetzungen enthalten mindestens einen Fluoroaluminiumsilikatglasfüllstoff (FAS-Füller) und/oder einen röntgenopaken Glasfüllstoff. Füllstoffhaltige Zusammensetzungen werden als Komposite bezeichnet.

Bevorzugte röntgenopake Glasfüllstoffe weisen die folgende Zusammensetzung auf (Gew.-%): SiO₂: 20-80; B₂O₃: 2-15; BaO oder SrO: 0-40; Al₂O₃: 2-20; CaO und/oder MgO: 0-20; Na₂O, K₂O, Cs₂O: je 0-10; WO₃: 0-20; ZnO: 0-20; La₂O₃: 0-10; ZrO₂: 0-15; P₂O₅: 0-30; Ta₂O₅, Nb₂O₅ oder Yb₂O₃: 0-5 und CaF₂ oder SrF₂: 0-10. Besonders bevorzugt sind röntgenopake Glasfüller mit der Zusammensetzung (Gew.-%): SiO₂: 50-75; B₂O₃: 2-15; BaO oder SrO: 2-35; Al₂O₃: 2-15; CaO und/oder MgO: 0-10 und Na₂O: 0-10.

Besonders bevorzugte FAS-Füller haben die folgende Zusammensetzung (Gew.-%): SiO₂: 20-35; Al₂O₃: 15-35; BaO oder SrO: 10-25; CaO: 0-20; ZnO: 0-15; P₂O₅: 5-20; Na₂O, K₂O, Cs₂O: je 0-10; und CaF₂: 0,5-20. Besonders bevorzugt sind FAS-Füller mit der Zusammensetzung (Gew.-%): SiO₂: 20-30; Al₂O₃: 20-30; BaO oder SrO: 10-25; CaO: 5-20; P₂O₅: 5-20; Na₂O: 0-10; und CaF₂: 5-20.

Alle Prozentangaben beziehen sich auf die Gesamtmasse des Glases, wobei die Komponenten mit Ausnahme von Fluor als Oxide berechnet werden, so wie es bei Gläsern und Glaskeramiken üblich ist.

Die erfindungsgemäßen Zusammensetzungen enthalten vorzugsweise 10 Gew.-% bis 80 Gew.-%, besonders bevorzugt 20 bis 75 Gew.-% und ganz besonders bevorzugt 30 bis 70 Gew.-% FAS-Füller und/oder röntgenopaken Glasfüller, jeweils bezogen auf die Gesamtmasse der Zusammensetzung.

Die FAS-Füller und röntgenopaken Glasfüller haben vorzugsweise eine mittlere Partikelgröße von 0,2 bis 20 µm und besonders bevorzugt von 0,4 bis 5 µm.

Wenn nicht anders angegeben, handelt es sich bei allen Partikelgrößen hierin um volumengemittelte Partikelgrößen (D50-Werte), d.h. 50 % des Gesamtvolumens, das alle Partikel gemeinsam besitzen, ist in Partikeln enthalten, die einen Durchmesser kleiner als der angegebene Wert aufweisen.

Die Partikelgrößenbestimmung im Bereich von 0,1 µm bis 1000 µm erfolgt vorzugsweise mittels statischer Lichtstreuung (SLS), beispielsweise mit einem statischen Laserstreuungs-Partikelgrößen-Analysator LA-960 (Horiba, Japan) oder mit einem Microtrac S100 Partikelgrößen-Analysator (Microtrac, USA). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320. Die Messung der Partikelgröße in einem Bereich von 1 nm bis 0,1 µm erfolgt vorzugsweise durch dynamische Lichtstreuung (DLS) von wässrigen Partikeldispersionen, vorzugsweise mit einem He-Ne-Laser mit einer Wellenlänge von 633 nm, bei einem Streuwinkel von 90° und bei 25°C, z.B. mit einem Malvern Zetasizer Nano ZS (Malvern Instruments, Malvern UK).

Im Fall aggregierter und agglomerierter Partikel kann die Primärteilchengröße anhand von TEM-Aufnahmen bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf ein 50 Å dickes Kupfergitter (Maschenweite 300 Mesh) aufgebracht, das mit Kohlenstoff beschichtet ist, und im Anschluss das Lösungsmittel verdampft. Die Partikel werden ausgezählt und der arithmetische Mittelwert berechnet.

Die Säurebehandlung der partikulären FAS- bzw. Glasfüllstoffe erfolgt dadurch, dass der Füllstoff mit Säure gewaschen wird, vorzugsweise durch folgendes Verfahren:
(i) Der partikuläre FAS- bzw. Glasfüllstoff wird in einer wässrigen Lösung einer organischen oder vorzugsweise anorganischen Säure dispergiert. Die Säure wird vorzugsweise in einer Konzentration von 0,1 bis 5 mol/I, besonders bevorzugt von 0,5 bis 3 mol/I eingesetzt.
(ii) Anschließend wird die Dispersion aus Schritt (i) gerührt, vorzugsweise für 0,5 bis 24 h, besonders bevorzugt für 1 bis 5 h.
(iii) Nach dem Rühren in Säurelösung wird der Füllstoff abgetrennt und mit deionisiertem Wasser gewaschen. Hierzu wird er vorzugsweise in Wasser dispergiert und für 1 bis 60 Minuten, besonders bevorzugt 2 bis 20 Minuten gerührt.
(iv) Nach dem Waschen wird der Füllstoff abgetrennt und getrocknet, vorzugsweise in einem Vakuumtrockenschrank im Feinvakuum bei 20 bis 80 °C, besonders bevorzugt bei 40 bis 60 °C. Der Füllstoff wird vorzugsweise bis zur Gewichtskonstanz getrocknet.
(v) Nach dem Trocknen wird der Füllstoff vorzugsweise einer optionalen Temperung unterworfen. Hierzu wird er vorzugsweise für 2 bis 12 h, besonders bevorzugt 4 bis 6 h, auf eine Temperatur erwärmt, die deutlich unterhalb der Glasübergangstemperatur des Füllstoffs liegt, vorzugsweise 200 bis 500 °C und besonders bevorzugt 300 bis 400 °C.

Bei dem erfindungsgemäßen Verfahren wird die zur Behandlung der Füllstoffe verwendete Säure nach der Säurebehandlung vorzugsweise vollständig von den Füllstoffen entfernt. Die Füllstoffe sind nicht mit einer Säure beschichtet.

Die Säurebehandlung kann ein- oder mehrfach wiederholt werden. Die Schritte (i) und (iii) werden vorzugsweise bei einer Temperatur im Bereich von 5 bis 50°C durchgeführt, besonders bevorzugt bei Raumtemperatur (23°C). Die Temperatur wird jeweils in der Lösung bzw. Dispersion gemessen.

Bevorzugt sind Säuren, die lösliche Salze mit Ca- Al-, Sr- und Ba-Ionen bilden. Besonders bevorzugt sind Ameisensäure, Essigsäure und ganz besonders Salzsäure, und Salpetersäure. Alternativ können zwar auch Säuren eingesetzt werden, die schwerlösliche Salze mit Ca-, Al-, Sr- oder Ba-Ionen bilden, wie z.B. Phosphorsäure, diese sind jedoch weniger bevorzugt. Unter schwerlöslichen Salzen werden Salze mit einer Löslichkeit von unter 0,1 g/l verstanden (in Wasser bei Raumtemperatur). Saure organische Monomere und saure organische Polymere sowie Persäuren und Flusssäure sind erfindungsgemäß als Säuren nicht geeignet.

Der Waschvorgang (iii) wird vorzugsweise 1 bis 5 Mal, besonders bevorzugt 3 Mal wiederholt, so dass die Säure vollständig von dem Füllstoff entfernt wird. Hierzu wird der Füllstoff im Anschluss an Schritt (iii) von dem Wasser abgetrennt und erneut in deionisiertem Wasser dispergiert und gerührt. Das Waschen wird so oft wiederholt, bis der pH-Werts des Wassers im letzten Waschschritt ≥ 5 ist.

Nach dem Waschen kann der Füllstoff einer weiteren Säurebehandlung und Wäsche unterworfen werden. Die Sequenz von Säurebehandlung und Waschen kann ein- oder mehrfach wiederholt werden.

Die erfindungsgemäßen Füllstoffe werden nach dem Trocken und ggf. Tempern vorzugsweise oberflächenmodifiziert, besonders bevorzugt silanisiert. Die Silanisierung erfolgt vorzugsweise mit einem radikalisch polymerisierbaren Silan, insbesondere mit z.B. 3-Methacryloyloxypropyltrimethoxysilan (MEMO). Die Füllstoffe sind gut mit den übrigen Bestandteilen der Kompositmaterialien mischbar.

Es wurde überraschend gefunden, dass die Säurebehandlung der Füllstoffe die Lagerstabilität der erfindungsgemäßen Zusammensetzungen deutlich verbessert und der Gehalt an säuregruppenhaltigen, radikalisch polymerisierbaren Monomeren nur langsam abnimmt. Die Zusammensetzungen zeigen auch nach längerer Aufbewahrung eine hohe Haftung an der Zahnhartsubstanz und insbesondere an Dentin. Die Säurebehandlung ermöglicht damit auf einfache Weise eine signifikante Eigenschaftsverbesserung von dentalen Kompositen mit selbsthaftenden Eigenschaften. Die erfindungsgemäßen Zusammensetzungen zeichnen sich nach der Härtung außerdem durch eine im Vergleich zu Glasionomerzementen hohe Transparenz aus.

Zusätzlich zu den genannten FAS- und röntgenopaken Glasfüllstoffen können die erfindungsgemäßen Zusammensetzungen weitere Füllstoffe enthalten.

Bevorzugte weitere Füllstoffe sind Metalloxide, besonders bevorzugt Mischoxide, die 60 bis 80 Gew.-% SiO₂ und mindestens eines der Metalloxide ZrO₂, Yb₂O₃, ZnO, Ta₂O₅, Nb₂O₅ und/oder La₂O₃, vorzugsweise ZrO₂, Yb₂O₃ und/oder ZnO enthalten, so dass sich die Gesamtmenge auf 100 % ergänzt. Mischoxide wie SiO₂-ZrO₂ sind z.B. durch hydrolytische Cokondensation von Metallalkoxiden zugänglich. Die Metalloxide haben vorzugsweise eine mittlere Partikelgröße von 0,05 bis 10 µm, besonders bevorzugt von 0,1 bis 5 µm. Das oder die Metalloxide werden vorzugsweise ebenfalls auf die oben beschriebene Weise mit Säure behandelt.

Weitere bevorzugte zusätzliche Füllstoffe sind pyrogene Kieselsäure oder Fällungskieselsäure mit einer Primärpartikelgröße von 0,01-0,15 µm sowie Quarz- oder Glaskeramikpulver mit einer Partikelgröße von 0,1 bis 15 µm, vorzugweise von 0,2 bis 5 µm, sowie Ytterbiumtrifluorid. Das Ytterbiumtrifluorid hat vorzugsweise eine Partikelgröße von 80 bis 900 nm und besonders bevorzugt 100 bis 300 nm. Diese Füller werden vorzugsweise in einer Menge von 0,1 bis 25 Gew.-%, besonders bevorzugt 0,2 bis 20 Gew.-% und ganz besonders bevorzugt in einer Menge von 0,3 bis 15 Gew.-% eingesetzt, jeweils bezogen auf die Gesamtmasse der Zusammensetzung.

Außerdem sind als weitere Füllstoffe sog. Kompositfüller bevorzugt. Diese werden auch als Isofüller bezeichnet. Hierbei handelt es sich um splitterförmige Polymerisate, die ihrerseits einen Füllstoff enthalten, vorzugsweise pyrogenes SiO₂, Glasfüller und/oder Ytterbiumtrifluorid. Bevorzugt sind Polymerisate auf der Basis von Dimethacrylaten. Zur Herstellung der Isofüller werden der oder die Füllstoffe z.B. in eine Dimethacrylatharzmatrix eingearbeitet, die so erhaltene Kompositpaste anschließend thermisch polymerisiert und dann gemahlen.

Ein erfindungsgemäß bevorzugter Kompositfüller lässt sich beispielsweise durch thermische Härtung einer Mischung von Bis-GMA (8,80 Gew.-%), UDMA (6,60 Gew.-%), 1,10-Decandioldimethacrylat (5,93 Gew.-%), Dibenzoylperoxid + 2,6-Di-tert.-butyl-4-methylphenol (zusammen 0,67 Gew.-%), Glasfüller (mittlere Korngröße 0,4 µm; 53,0 Gew.-%) und YbF₃ (25,0 Gew.-%) und anschließendes Vermahlen des gehärteten Materials auf die gewünschte Korngröße herstellen. Alle Prozentangaben beziehen sich auf die Gesamtmasse des Kompositfüllers.

Als weitere Füllstoffe können auch sog. inertisierte Füllstoffe eingesetzt werden. Dabei handelt es sich um Glasfüllstoffe, deren Oberfläche mit einer Diffusionsbarriereschicht, z.B. auf Sol-Gel-Basis, oder mit einer Polymerschicht, z.B. aus PVC, beschichtet ist. Bevorzugt sind die in der EP 2 103 296 A1 beschrieben Füllstoffe.

Zur Verbesserung des Verbundes zwischen Füllstoff und Matrix sind die Füllstoffe vorzugsweise mit methacrylatfunktionalisierten Silanen, wie z.B. 3-Methacryloyloxypropyltrimethoxysilan, oberflächenmodifiziert.

Die erfindungsgemäßen Zusammensetzungen enthalten vorzugsweise 0,1 bis 25 Gew.-%, bevorzugt 1 bis 20 Gew.-% und besonders bevorzugt 2 bis 15 Gew.-% eines oder mehrerer weiterer Füllstoffe, vorzugsweise eines oder mehrerer Metalloxide, pyrogene Kieselsäure und/oder Fällungskieselsäure, jeweils bezogen auf die Gesamtmasse der Zusammensetzung.

Die erfindungsgemäßen Zusammensetzungen enthalten mindestens ein radikalisch polymerisierbares Monomer, vorzugsweise ein oder mehrere mono- und/oder polyfunktionelle Monomere. Unter polyfunktionellen Monomeren werden Verbindungen mit zwei oder mehr, vorzugsweise 2 bis 4 und insbesondere 2 radikalisch polymerisierbaren Gruppen verstanden. Monofunktionelle Monomere weisen dementsprechend nur eine radikalisch polymerisierbare Gruppe auf. Polyfunktionelle Monomere haben vernetzende Eigenschaften und werden daher auch als Vernetzermonomere bezeichnet. Bevorzugte radikalisch polymerisierbare Gruppen sind (Meth)acrylat-, (Meth)acrylamid- und Vinylgruppen.

Erfindungsgemäß wird zwischen säuregruppenhaltigen Monomeren und Monomeren unterschieden, die keine Säuregruppen enthalten. Die erfindungsgemäßen Zusammensetzungen enthalten mindestens ein Monomer ohne Säuregruppen und mindestens ein Monomer und/oder Oligomer mit Säuregruppen. Erfindungsgemäß sind Zusammensetzungen bevorzugt, die Monomere mit und Monomere ohne Säuregruppe in einem Gewichtsverhältnis von 1:5 bis 1:36, besonders bevorzugt von 1:6 bis 1:25 und ganz besonders bevorzugt von 1:7 bis 1:20 enthalten.

### Monomere ohne Säuregruppe

Bevorzugt sind Zusammensetzungen, die mindestens ein (Meth)acrylat enthalten, besonders bevorzugt mindestens ein monofunktionelles oder polyfunktionelles Methacrylat und ganz besonders bevorzugt mindestens ein monofunktionelles oder difunktionelles Methacrylat oder eine Mischung davon.

Bevorzugte monofunktionelle (Meth)acrylate sind Benzyl-, Tetrahydrofurfuryl- oder Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecanmethyl(meth)-acrylat, 2-(2-Biphenyloxy)ethyl(meth)acrylat. Besonders geeignet sind CMP-1E und MA-836.

Gemäß einer Ausführungsform enthalten die erfindungsgemäßen Zusammensetzungen vorzugsweise mindestens ein funktionalisiertes monofunktionelles (Meth)acrylat. Unter funktionalisierten Monomeren werden solche Monomere verstanden, die neben mindestens einer radikalisch polymerisierbaren Gruppe mindestens eine funktionelle Gruppe tragen, vorzugsweise eine Hydroxylgruppe. Bevorzugte funktionalisierte Mono(meth)acrylate sind 2-Hydroxyethyl- und Hydroxyethylpropyl(methacrylat) sowie 2-Acetoxyethylmethacrylat. Besonders bevorzugt ist Hydroxyethylmethacrylat. Die unten genannten säuregruppenhaltigen Monomere sind keine funktionalisierten Monomere im Sinne der Erfindung.

Bevorzugte di- und polyfunktionelle (Meth)acrylate sind Bisphenol-A-dimethacrylat, Bis-GMA (ein Additionsprodukt aus Methacrylsäure und Bisphenol-A-diglycidylether), ethoxy- oder propoxylierte Bisphenol-A-dimethacrylate, wie z.B. das Bisphenol-A-dimethacrylat SR-348c (Sartomer) mit 3 Ethoxygruppen oder 2,2-Bis[4-(2-methacryl-oyloxypropoxy)phenyl]propan, Urethane aus 2-(Hydroxymethyl)acrylsäuremethylester und Diisocyanten, wie 2,2,4-Trimethylhexamethylendiisocyanat oder Isophorondiisocyanat, UDMA (ein Additionsprodukt aus 2-Hydroxyethylmethacrylat und 2,2,4-Trimethylhexamethylen-1,6-diisocyanat), Tetramethylxylylendiurethanethylenglykoldi(meth)acrylat bzw. Tetramethylxylylendiurethan-2-methylethylenglykoldi-(meth)acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.0^{2,6}]decan (DCP), Polyethylenglycol- oder Polypropylenglycoldimethacrylate, wie z.B. Polyethylenglycol-200-dimethacrylat oder Polyethylenglycol-400-dimethacrylat (PEG-200- oder PEG-400-DMA) oder 1,12-Dodecandioldimethacrylat. Besonders bevorzugt sind Bis-GMA, UDMA, V-380, Triethylenglycoldimethacrylat (TEGDMA) und PEG-400-DMA (NK-Ester 9G).

Das Monomer Tetramethylxylylendiurethanethylenglykoldi(meth)acrylat bzw. Tetra-methylxylylendiurethan-2-methylethylenglykoldiurethandi(meth)acrylat (V380) weist die folgende Formel auf:

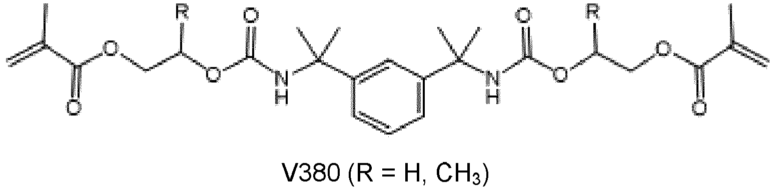

In der gezeigten Formel sind die Reste R unabhängig voneinander jeweils H oder CH₃, wobei die Reste die gleiche Bedeutung oder unterschiedliche Bedeutungen haben können. Vorzugsweise wird eine Mischung eingesetzt, die Moleküle, in denen beide Reste H sind, Moleküle, in denen beide Reste CH₃ sind, und Moleküle enthält, in denen ein Rest H und der andere Rest CH₃ ist, wobei das Verhältnis von H zu CH₃ vorzugsweise 7:3 ist. Eine solche Mischung ist beispielsweise durch Reaktion von 1,3-Bis(1-isocyanato-1-methylethyl)benzol mit 2-Hydroxypropylmethacrylat und 2-Hydroxyethylmethacrylat erhältlich.

Weitere bevorzugte difunktionelle Monomere sind radikalisch polymerisierbare Pyrrolidone, wie z.B. 1,6-Bis(3-vinyl-2-pyrrolidonyl)-hexan, oder kommerziell zugängliche Bisacrylamide wie Methylen- oder Ethylenbisacrylamid, sowie Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Besonders bevorzugt ist N,N'-Diethyl-1,3-bis(acrylamido)propan (V-392). Diese Monomere zeichnen sich durch eine hohe Hydrolysestabilität aus.

### Säuregruppenhaltige Monomere und Oligomere

Die erfindungsgemäßen Zusammensetzungen enthalten mindestens ein saures, radikalisch polymerisierbares Monomer und/oder mindestens ein saures Oligomer. Unter sauren Monomeren und Oligomeren werden Monomere bzw. Oligomere verstanden, die mindestens eine Säuregruppe enthalten, vorzugsweise eine Phosphorsäureester-, Phosphonsäure- oder Carboxygruppe. Saure Monomere und Oligomere werden hierin auch als Haftkomponente bzw. Haftmonomere oder Haftoligomere bezeichnet. Erfindungsgemäß sich solche Zusammensetzungen besonders bevorzugt, die mindestens ein stark saures Monomer enthalten. Unter stark sauren Monomeren werden Monomere mit einem pKa-Wert von 0,5 bis 4,0, besonders bevorzugt 1,0 bis 3,5 und ganz besonders bevorzugt 1,5 bis 2,5 bei Raumtemperatur verstanden.

Geeignete säuregruppenhaltige Monomere sind COOH-gruppenhaltige polymerisationsfähige Monomere, vorzugsweise mit einem pKa-Wert im Bereich von 2,0 bis 4,0. Bevorzugt sind 4-(Meth)acryloyloxyethyltrimellitsäureanhydrid, 10-Methacryloyloxydecylmalonsäure, N-(2-Hydroxy-3-methacryloyloxypropyl)-N-phenylglycin und 4-Vinylbenzoesäure. Methacrylsäure (pKa = 4,66) ist aufgrund ihrer geringen Haftung auf der Zahnhartsubstanz ausgeschlossen.

Bevorzugte säuregruppenhaltige Monomere sind Phosphorsäureester- und Phosphonsäuremonomere, vorzugsweise mit einem pKa-Wert im Bereich von 0,5 bis 3,5. Besonders bevorzugte sind 2-Methacryloyloxyethylphenyl-hydrogenphosphat, 10-Methacryloyloxydecyl-dihydrogenphosphat (MDP) Glycerindimethacrylatdihydrogenphosphat oder Dipentaerythritpentamethacryloyloxyphosphat. 4-Vinylbenzylphosphonsäure, 2-[4-(Dihydroxyphosphoryl)-2-oxa-butyl]-acrylsäure oder hydrolysestabile Ester, wie z.B. 2-[4-(Dihydroxyphosphoryl)-2-oxa-butyl]-acrylsäure-2,4,6-trimethylphenylester. Ganz besonders bevorzugt sind MDP, 2-Methacryloyloxyethylphenyl-hydrogenphosphat und Glycerindimethacrylatdihydrogenphosphat.

Unter Oligomeren werden Polymere mit einem Polymerisationsgrad Pₙ von 2 bis 100 verstanden (Pₙ = Mₙ / Mᵤ; Mₙ: zahlenmittlere Polymermolmasse, Mᵤ: Molmasse der Monomereinheit). Saure, radikalisch polymerisierbare Oligomere weisen mindestens eine Säuregruppe, vorzugsweise Carboxygruppe, und mindestens eine radikalisch polymerisierbare Gruppe, vorzugsweise mindestens eine (Meth)acrylat- und insbesondere mindestens eine Methacrylatgruppe, auf.

Erfindungsgemäß bevorzugte säuregruppenhaltige Oligomere sind oligomere Carbonsäuren, wie z.B. Polyacrylsäure, vorzugsweise mit einer zahlenmittleren Molmasse Mₙ kleiner 7200 g/mol, bevorzugt kleiner 7000 g/mol und besonders bevorzugt kleiner 6800 g/mol, wobei Mₙ bevorzugt in einem Bereich von 800 bis 7200 g/mol, besonders bevorzugt von 500 bis 7000 g/mol und ganz besonders bevorzugt von 500 bis 6800 g/mol liegt. Besonders bevorzugt sind oligomere Carbonsäuren mit (Meth)acrylatgruppen. Diese sind z.B. durch Umsetzung von oligomerer Polyacrylsäure mit Glycidylmethacrylat oder 2-Isocyanatoethylmethacrylat erhältlich.

Wenn nicht anders angegeben, handelt es sich hierin bei der Molmasse von Oligomeren und Polymeren um die zahlenmittlere Molmasse, deren Absolutwerte man mit den bekannten Methoden der Gefrierpunktserniedrigung (Kryoskopie), der Siedepunktserhöhung (Ebullioskopie) oder aus der Erniedrigung des Dampfdrucks (Dampfdruckosmometrie) bestimmen kann. Vorzugsweise wird die zahlenmittlere Molmasse von Oligomeren und Polymeren mittels Gel-Permeations-Chromatographie (GPC) bestimmt. Dabei handelt es sich um eine Relativmethode bei der die Moleküle aufgrund ihrer Größe, genauer gesagt aufgrund ihres hydrodynamischen Volumens, getrennt werden. Die Bestimmung der absoluten Molmasse erfolgt durch Kalibrierung mit bekannten Standards.

Die erfindungsgemäßen Zusammensetzungen enthalten darüber hinaus vorzugsweise auch Wasser. Es wurde gefunden, dass ein Wassergehalt von 1 bis 7 Gew.-%, besonders bevorzugt 1 bis 5 Gew.-%, jeweils bezogen auf die Gesamtmasse der Zusammensetzung, eine Verbesserung der Haftwirkung an Dentin und Zahnschmelz bewirkt.

Die erfindungsgemäßen Zusammensetzungen enthalten außerdem mindestens einen Initiator zur Initiierung der radikalischen Polymerisation, vorzugsweise einen Photoinitiator. Bevorzugte Photoinitiatoren sind Benzophenon, Benzoin sowie deren Derivate, α-Diketone oder deren Derivate, wie 9,10-Phenanthrenchinon, 1-Phenyl-propan-1,2-dion, Diacetyl und 4,4'-Dichlorbenzil. Besonders bevorzugt werden Campherchinon (CC) und 2,2-Dimethoxy-2-phenyl-acetophenon und ganz besonders bevorzugt α-Diketone in Kombination mit Aminen als Reduktionsmittel, wie z.B. 4-(Dimethylamino)-benzoesäureethylester (EDMAB), N,N-Dimethylaminoethylmethacrylat, N,N-Dimethyl-sym.-xylidin oder Triethanolamin verwendet. Weiter bevorzugt sind Norrish-Typ-I-Photoinitiatoren, vor allem Acyl- oder Bisacylphosphinoxide und ganz besonders Monoacyltrialkyl-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin^{®}), Tetrabenzoylgerman oder Tetrakis(o-methylbenzoyl)german. Dabei lassen sich auch Mischungen der verschiedenen Photoinitiatoren einsetzen, wie z.B. Bis(4-methoxybenzoyl)diethylgerman oder Tetrakis(o-methylbenzoyl)german in Kombination mit Campherchinon und 4-Dimethylaminobenzoesäureethylester.

Weiter bevorzugt sind Zusammensetzungen, die einen Redoxinitiator zur Initiierung der radikalischen Polymerisation enthalten, vorzugsweise einen Redoxinitiator auf der Basis eines Oxidationsmittels und eines Reduktionsmittel. Bevorzugte Oxidationsmittel sind Peroxide und insbesondere Hydroperoxide. Ein besonders bevorzugtes Peroxid ist Benzoylperoxid. Bevorzugte Hydroperoxide sind die in EP 3 692 976 A1 offenbarten geruchsarmen Cumolhydroperoxid-Derivate, die in EP 21315089.9 offenbarten oligomeren CHP-Derivate und insbesondere 4-(2-Hydroperoxypropan-2-yl)phenyl¬propionat und Cumolhydroperoxid (CHP).

Bevorzugte Reduktionsmittel zur Kombination mit Peroxiden sind tertiäre Amine, wie N,N-Dimethyl-p-toluidin, N,N-Dihydroxyethyl-p-toluidin, p-Dimethylaminobenzoesäureethylester oder andere aromatische Dialkylamine, Ascorbinsäure, Sulfinsäuren, Thiole und/oder Hydrogensilane.

Bevorzugte Reduktionsmittel zur Kombination mit Hydroperoxiden sind Thioharnstoffderivate, insbesondere die in der EP 1 754 465 A1 in Absatz [0009] aufgeführten Verbindungen. Besonders bevorzugt sind Methyl-, Ethyl-, Allyl-, Butyl-, Hexyl-, Octyl-, Benzyl-, 1,1,3-Trimethyl-, 1,1-Diallyl, 1,3-Diallyl-, 1-(2-Pyridyl-2-thioharnstoff, Acetyl-, Propanoyl-, Butanoyl-, Pentanoyl-, Hexanoyl-, Heptanoyl-, Octanoyl, Nonanoyl, Decanoyl, Benzoylthioharnstoff und Mischungen davon. Ganz besonders bevorzugt sind Acetyl-, Allyl-, Pyridyl- und Phenylthioharnstoff sowie Hexanoylthioharnstoff und Mischungen davon sowie polymerisationsfähige Thioharnstoff-Derivate, wie z.B. *N*-(2-Methacryloyloxyethoxysuccinoyl)-thioharnstoff und *N*-(4-Vinylbenzoyl)-thioharnstoff). Außerdem kann vorteilhaft eine Kombination von einem oder mehreren der genannten Thioharnstoffderivate mit einem oder mehreren Imidazolen eingesetzt werden. Bevorzugte Imidazole sind 2-Mercapto-1-methylimidazol oder 2-Mercaptobenzimidazol.

Die erfindungsgemäßen Zusammensetzungen können neben mindestens einem Hydroperoxid und mindestens einem Thioharnstoffderivat außerdem zusätzlich mindestens eine Übergangsmetallverbindung zur Beschleunigung der Härtung enthalten. Erfindungsgemäß geeignete Übergangsmetallverbindungen sind insbesondere Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Zusammensetzungen, die mindestens eine Kupferverbindung enthalten, sind besonders geeignet. Die Übergansmetallverbindungen werden vorzugsweise in katalytische Mengen eingesetzt, besonders bevorzugt in einer Menge von 10 bis 200 ppm. Diese führen zu keiner Verfärbungen der Dentalmaterialien. Aufgrund der guten Monomerlöslichkeit werden die Übergangsmetalle vorzugsweise in Form ihrer Acetylacetonate, 2-Ethylhexanoate oder THF-Addukte eingesetzt. Bevorzugt sind weiterhin ihre Komplexe mit mehrzähnigen Liganden wie z.B. 2-(2-Amino-ethylamino)ethanol, Triethylentetramin, Dimethylglyoxim, 8-Hydroxychinolin, 2,2'-Bipyridin oder 1,10-Phenanthrolin. Ein erfindungsgemäß besonders geeigneter Initiator ist eine Mischung von Cumolhydroperoxid (CHP) mit mindestens einem der oben genannten Thioharnstoffderivate und Kupfer(II)-acetylacetonat. Erfindungsgemäß sind Zusammensetzungen bevorzugt, die keine Vanadiumverbindungen enthalten.

Die erfindungsgemäßen Zusammensetzungen enthalten vorzugsweise keine Barbitursäure oder Barbitursäure-Derivate wie 1,3,5-Trimethylbarbitursäure, 1-Benzyl-5-phenylbarbitursäure, 5-Butylbarbitursäure oder 1-Cylohexyl-5-ethylbarbitursäure. Zusammensetzungen, die Barbiturate enthalten, weisen eine unbefriedigende Lagerstabilität auf, weil Barbiturate durch Oxidation mit Luftsauerstoff polymerisationsauslösende Radikale bilden. Außerdem haben Barbiturate nachteilige physiologische Wirkungen wie Bradykardie, Hypotonie oder Blutstörungen.

Gemäß einer bevorzugten Ausführungsform enthalten die erfindungsgemäßen Zusammensetzungen vorzugsweise zusätzlich ein Maskierungsmittel. Erfindungsgemäß bevorzugte Maskierungsmittel sind Ethylendiamintetraessigsäure (EDTA) und deren Dinatrium-Salz (Dinatrium-ethylendiamin-tetraacetat), Nitrilotriessigsäure, Diethylentriaminpentaessigsäure, Tetranatriumiminodisuccinat und das Trinatriumsalz der Methylglycindiessigsäure. Besonders bevorzugt ist EDTA. Weiterhin eingeschlossen sind auch polymerisationsfähige Derivate der voranstehend genannten Maskierungsmittel. Unter polymerisationsfähigen Derivaten werden Maskierungsmittel mit radikalisch polymerisierbaren Gruppen verstanden. Bevorzugte radikalisch polymerisierbare Maskierungsmittel sind EDTA-Derivate, die polymerisationsfähige (Meth)acryl- oder Methacrylamidgruppen tragen. Besonders bevorzugt sind die in der DE 10 2005 022 172 A1 offenbarten polymerisierbaren EDTA-Derivate, in denen EDTA kovalent mit ethylenisch ungesättigten Monomeren verknüpft ist, sowie die in der EP 2 065 363 A1 offenbarten Alkylendiamin-N,N,N',N'-tetraessigsäure-(meth)-acrylamide. Das Maskierungsmittel kann in gelöster oder vorzugsweise ungelöster Form vorliegen.

Das oder die Maskierungsmittel werden vorzugsweise in einer Gesamtmenge von 0,5 bis 6,0 Gew.-%, besonders bevorzugt 0,7 bis 5 Gew.-% und ganz besonders bevorzugt 1,0 bis 4,0 Gew.-% zugegeben. Alle Prozentangaben hierin beziehen sich, wenn nicht anders angegeben, auf die Gesamtmasse der Zusammensetzung.

Die erfindungsgemäßen Zusammensetzungen können außerdem zusätzlich weitere Additive enthalten, vor allem Stabilisatoren, Farbmittel, Phasentransferkatalysatoren, mikrobizide Wirkstoffe, fluoridionenabgebende Additive, wie z.B. Fluorid-Salze, insbesondere NaF oder Ammoniumfluorid, oder Fluorsilane, optische Aufheller, Weichmacher und/oder UV-Absorber.

Erfindungsgemäß sind solche Zusammensetzungen besonders bevorzugt, die die folgenden Bestandteile enthalten:
a) 10 bis 80 Gew.-%, bevorzugt 20 bis 75 Gew.-% und besonders bevorzugt 30 bis 70 Gew.-% mindestens eines säurebehandelten FAS- und/oder Glasfüllstoffs,
b) ggf. 0,1 bis 25 Gew.-%, bevorzugt 1 bis 20 Gew.-% und besonders bevorzugt 2 bis 15 Gew.-% eines oder mehrerer weiterer Füllstoffe,
c) 1 bis 15 Gew.-%, bevorzugt 2 bis 12 Gew.-% und besonders bevorzugt 3 bis 10 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,1 bis 8 Gew.-%, bevorzugt 0,5 bis 6 Gew.-% und besonders bevorzugt 1 bis 5 Gew.-% eines Initiators für die radikalische Polymerisation,
h) 0 bis 20 Gew.-%, bevorzugt 0,2 bis 10 Gew.-% und besonders bevorzugt 1 bis 7 Gew.-% an Wasser und
i) 0,01 bis 5 Gew.-%, bevorzugt 0,1 bis 3 Gew.-% und besonders bevorzugt 0,1 bis 2 Gew.-% eines oder mehrerer Additive, jeweils bezogen auf die Gesamtmasse der Zusammensetzung.

Der Initiator kann ein Redoxinitiator, ein Photoinitiator oder ein Initiator für die duale Härtung sein. Die genannten Mengenangaben enthalten sämtliche Initiatorbestandteile, d.h. die Initiatoren selbst und ggf. Reduktionsmittel, Übergangsmetallverbindung etc. Erfindungsgemäß sind Zusammensetzungen bevorzugt, die mindestens einen Redoxinitiator oder mindestens einen Redoxinitiator und mindestens einen Photoinitiator enthalten.

Zusammensetzungen, die einen Redoxinitiator enthalten, werden auch als selbsthärtend bezeichnet. Sie werden vorzugsweise in der Form von zwei räumlich getrennten Komponenten, d.h. als 2-Komponenten-System (2K System), eingesetzt. Oxidations- und Reduktionsmittel werden dabei in separate Komponenten der Zusammensetzung eingebarbeitet. Eine Komponente, die sog. Katalysatorpaste, enthält das Oxidationsmittel, vorzugsweise ein Peroxid oder Hydroperoxid, und die zweite Komponente, die sog. Basis-Paste, das entsprechende Reduktionsmittel und ggf. einen Photoinitiator und ggf. katalytische Mengen einer Übergansmetallverbindung. Die Polymerisation wird durch das Mischen der Komponenten gestartet. Zusammensetzungen, die sowohl einen Redox- als auch einen Photoinitiator enthalten, werden als dualhärtend bezeichnet.

Falls die Zusammensetzungen ein Maskierungsmittel enthalten, wird dieses bevorzugt der Komponente zugesetzt, in der sich das stark saure Haftmonomer, der FAS-Füllstoff und/oder der röntgenopake Glasfüllstoff befinden.

Erfindungsgemäß sind 2-Komponenten-Systeme bevorzugt. Sie sind vorzugsweise selbsthärtend oder dualhärtend. Die Pasten werden kurz vor der Anwendung vorzugsweise mit einer Doppelschubspritze miteinander gemischt.

Die Katalysatorpaste weist vorzugsweise die folgende Zusammensetzung auf:
a) 10 bis 80 Gew.-%, bevorzugt 20 bis 75 Gew.-% und besonders bevorzugt 30 bis 70 Gew.-% mindestens eines säurebehandelten FAS- und/oder Glasfüllstoffs,
b) ggf. 0,1 bis 25 Gew.-%, bevorzugt 1 bis 20 Gew.-% und besonders bevorzugt 2 bis 15 Gew.-% eines oder mehrerer weiterer Füllstoffe,
c) 2 bis 30 Gew.-%, bevorzugt 4 bis 24 Gew.-% und besonders bevorzugt 6 bis 20 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,01 bis 16 Gew.-%, bevorzugt 0,2 bis 12 Gew.-% und besonders bevorzugt 0,5 bis 10 Gew.-% mindestens eines Peroxids und/oder Hydroperoxids ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive, jeweils bezogen auf die Gesamtmasse der Katalysatorpaste.

Die Basis-Paste weist vorzugsweise folgende Zusammensetzung auf:
a) 10 bis 80 Gew.-%, bevorzugt 20 bis 75 Gew.-% und besonders bevorzugt 30 bis 70 Gew.-% mindestens eines säurebehandelten FAS- und/oder Glasfüllstoffs,
b) 0,1 bis 25 Gew.-%. bevorzugt 1 bis 20 Gew.-% und besonders bevorzugt 2 bis 15 Gew.-% eines oder mehrerer weiterer Füllstoffe,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,01 bis 16 Gew.-%, bevorzugt 0,3 bis 12 Gew.-% und besonders bevorzugt 1 bis 10 Gew.-% mindestens eines geeigneten Reduktionsmittels und ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive, jeweils bezogen auf die Gesamtmasse der Basis-Paste.

Zur Anwendung werden die Katalysator- und Basis-Paste vorzugsweise in etwa gleichen Anteilen miteinander gemischt. Sie eignen sich daher besonders zur Anwendung mit einer Doppelschubspritze.

Doppelschubspritzen weisen zwei getrennte zylindrische Kammern zur Aufnahme von Basis- und Katalysatorpaste auf. Die Komponenten werden mit zwei miteinander verbundenen Kolben gleichzeitig aus den Kammern herausgedrückt und vorzugsweise durch eine Mischkanüle gepresst und darin miteinander gemischt. Zum Herausdrücken der Pasten kann die Spritze in einen sogenannten Handdispenser eingesetzt werden, der die Handhabung der Spritzen erleichtert.

Die erfindungsgemäßen Zusammensetzungen zeichnen sich durch eine hohe Lagerstabilität und eine verbesserte Transparenz, vorzugsweise größer 10 %, und gute Selbsthaftung auf Schmelz/Dentin aus. Sie eignen sich besonders als Dentalwerkstoffe zur intraoralen Anwendung durch den Zahnarzt zur Restauration geschädigter Zähne (therapeutische Anwendung), insbesondere als dentale Zemente, Beschichtungs- oder Verblendmaterialien, Füllungskomposite und ganz besonders als Befestigungszemente. Die Transparenz wird auf die in den Ausführungsbeispielen beschriebene Weise bestimmt.

Zur Behandlung geschädigter Zähne werden diese vorzugsweise in einem ersten Schritt durch den Zahnarzt präpariert. Anschließend wird mindestens eine erfindungsgemäße Zusammensetzung auf oder in den präparierten Zahn appliziert. Danach kann die Zusammensetzung direkt gehärtet werden, vorzugsweise durch Bestrahlen mit Licht einer geeigneten Wellenlänge, beispielsweise bei der Versorgung von Kavitäten. Alternativ wird eine Dentalrestauration, beispielsweise ein Inlay, Onlay, Veneer, eine Krone, Brücke, ein Gerüst oder eine Dentalkeramik, in den präparierten Zahn eingebracht oder darauf aufgebracht. Die anschließende Härtung der Zusammensetzung erfolgt vorzugsweise durch Licht und/oder durch Selbsthärtung. Die Dentalrestauration wird hierbei am Zahn befestigt.

Die erfindungsgemäßen Zusammensetzungen können auch als extraorale Werkstoffe (nicht therapeutisch) eingesetzt werden, beispielsweise bei der Herstellung oder Reparatur von Dentalrestaurationen. Sie eignen sich zudem als Materialien zur Herstellung und Reparatur von Inlays, Onlays, Kronen oder Brücken.

Zur Herstellung von Dentalrestaurationen wie Inlays, Onlays, Kronen oder Brücken, wird mindestens eine erfindungsgemäße Zusammensetzung auf an sich bekannte Weise zu der gewünschten Dentalrestauration geformt und dann gehärtet. Die Härtung kann durch Licht, Selbsthärtung oder vorzugsweise thermisch erfolgen.

Bei der Reparatur von Dentalrestaurationen werden die erfindungsgemäßen Zusammensetzungen auf die zu reparierende Restauration aufgebacht, beispielsweise um Lücken auszubessern oder um Bruchstücke zu verkleben, und anschließend gehärtet.

Die Erfindung wird im Folgenden anhand von Figuren und Beispielen näher erläutert.

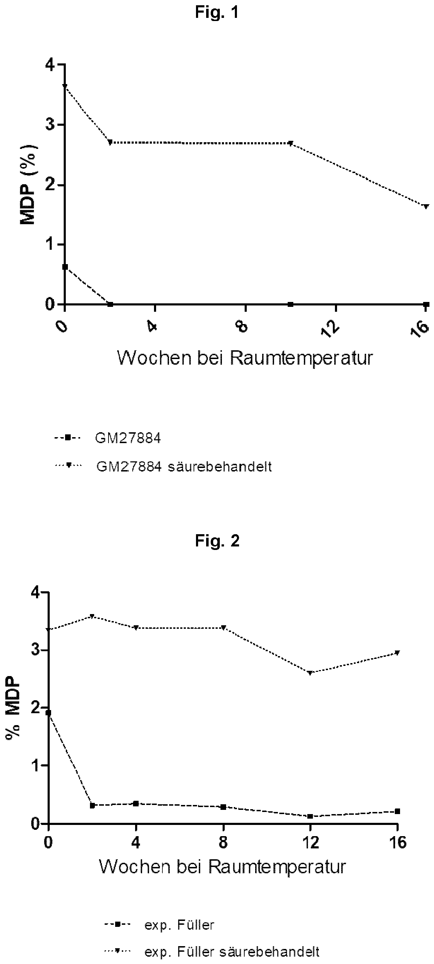

Figur 1 und Figur 2 zeigen jeweils die Abnahme der Konzentration des sauren Monomers MDP in Abhängigkeit von der Lagerzeit in Kompositpasten mit einem säurebehandelten (- -▼- -) oder einem nicht säurebehandelten Glasfüllstoff (- -■- -).

### Beispiel 1

### Herstellung von säurebehandelten Füllstoffen (allgemeine Vorschrift)

In zwei Zentrifugenkunststoffgefäßen mit einem Füllvolumen von jeweils 1 L werden je 150 g des zu behandelten Füllstoffs und 350 g 1,0 molare Salzsäure eingefüllt und 1 h bei Raumtemperatur auf einer Magnetrührplatte gerührt. Nach dem Entfernen der Magnetrührer werden die Mischungen in einer Zentrifuge (Hettich Silenta RS) für 5 min bei 3000 Umdrehungen pro min zentrifugiert, wobei sich der Füllstoff absetzt. Die Flüssigkeit wird abgetrennt und eine Probe der Flüssigkeit für die Röntgenfluoreszens (XRF)-Analyse genommen. Der pH-Wert der Flüssigkeit liegt bei 1-2. Anschließend wird der abgetrennte Füllstoff in 400 ml deionisiertem Wasser dispergiert und die Dispersion erneut für 5 min bei 3000 Umdrehungen pro min zentrifugiert. Dann wird die Waschlösung durch Dekantieren abgetrennt. Der Waschvorgang wird so oft wiederholt, bis der pH-Wert im letzten Waschvorgang auf 5 oder mehr ansteigt (ca. dreimal). Nach dem Waschen wird der säurebehandelte Füllstoff Im Vakuumtrockenschrank bei 50 °C bis zur Gewichtskonstanz getrocknet und dann silanisiert. Zur Silanisierung wird der Füllstoff (185 g) mit 12 g 3-Methacryloyloxypropyltrimethoxysilan (Silan A-174, Sigma Aldrich) versetzt und anschließend für 15 min gemischt (Turbola-Mischer, Willy A. Bachofen AG). Danach werden 5 g deionisiertes Wasser zugegeben und nochmals für 15 min gemischt. Anschließend wird der Füllstoff durch ein 90-µm-Kunststoffsieb gesiebt, für 24 h ruhen gelassen und dann für 3 Tage im Trockenschrank bei 50 °C getrocknet bis kein freies Silan mehr nachweisbar ist (Gaschromatographie).

### Beispiel 2

### Untersuchung der Lagerstabilität von Kompositen auf der Basis des Füllstoffs GM 27884 (mit und ohne Säurebehandlung)

Ein röntgenopaker, dentaler Glasfüllstoff (GM 27884, Firma Schott, mittlere Partikelgröße 1 µm, spezifische Oberfläche (BET DIN ISO 9277) 3,9 m²/g, Zusammensetzung (Gew.-%): Al₂O₃: 10, B₂O₃: 10, BaO: 25 und SiO₂: 55) wurde auf die in Beispiel 1 beschriebene Weise mit Säure behandelt und silanisiert. In der sauren Behandlungslösung wurden mit XRF-Analyse vor allem Aluminium- und Bariumionen nachgewiesen. Für Vergleichszwecke wurde ein Teil des Füllstoffs ohne vorherige Säurebehandlung silanisiert.

Mit dem säurebehandelten Füllstoff (Paste 1) und unbehandeltem Füllstoff (Paste 2) wurden Kompositpasten mit der folgenden Zusammensetzung hergestellt (Gew.-%): Füllstoff: 65,39, 10-Methacryloyloxydecyldihydrogenphosphat (MDP, Orgentis): 3,67, Triethylenglycoldimethacrylat (TEGDMA): 9,52, NK-Ester 9G (Polyethylenglycol-400-dimethacrylat, Kowa Europa GmbH): 2,12, V-392 (N,N'-Diethyl-1,3-bis(acrylamido)-propan, Ivoclar Vivadent AG): 13,04, BHT (2,6-Di-*tert*-butyl-*p*-kresol): 0,04 und deionisiertes Wasser: 6,23.

Die Pasten wurden bei Raumtemperatur gelagert und im Abstand von einigen Wochen der Gehalt an MDP mittels HPLC bestimmt. Für die HPLC-Messung wurde ein Gerät HPLC Ultimate 3000 (ThermoFisher Scientific) mit einer Säule 125x4 Nucleodur 100-5 C18ec mit UV/VIS-Detektor (220 nm) verwendet. Dabei wurde die Probe in Methanol gelöst und mit 0,01 mol/I H₃PO₄ in Wasser (A), Methanol (B) und Acetonitril (C) gemäß dem folgenden Programm eluiert. Die Ergebnisse sind in Fig. 1 dargestellt.

**Gradientenprogramm:**

| Zeit (min) | % A | % B | % C |
|---|---|---|---|
| 0 | 40 % | 40 % | 20 % |
| 6 | 40 % | 40 % | 20 % |
| 20 | 0% | 0% | 100 % |
| 24 | 0 % | 0 % | 100 % |

Die in Fig. 1 gezeigten Ergebnisse belegen eine deutlich verbesserte Lagerstabilität der Paste auf Basis des mit Säure behandelten Füllstoffs. In der Paste mit dem unbehandelten Füllstoff war das MDP nach nur 1 Woche nicht mehr nachweisbar.

### Beispiel 3

### Herstellung von dualhärtenden selbsthaftenden Kompositzementen

Es wurden dualhärtende Kompositzemente hergestellt. Jeder Zement umfasste ein Katalysator- und eine Basispaste. Die Zusammensetzungen der Pasten sind in den Tabellen 1 und 2 angegeben. Der zur Herstellung der Katalysatorpaste 1 verwendete Glasfüllstoff wurde auf die in Beispiel 1 beschriebene Weise mit Säure behandelt. Alle Füllstoffe wurden silanisiert. Es wurde die Dentinhaftung in Abhängigkeit von der Lagerzeit bestimmt. Für die Untersuchung der Dentinhaftung wurden Rinderzähne mit einem additionsvernetzenden Vinyl-Polysiloxan (Firma Dreve) so in einen Kunststoffzylinder eingebettet, dass sich das Dentin und der Kunststoff in einer Ebene befanden. Die mit Schleifpapier (Körnung 400) geschliffenen Zahnoberflächen wurden mit handwarmem Wasser gespült und auf 37° C vortemperiert. Die Dentinoberflächen wurden trockengetupft, die Katalysatorpasten jeweils mit der zugehörigen Basispaste im Verhältnis 1:1 gemischt und anschließend auf die Zahnoberfläche aufgebracht. Gleichzeitig wurde die Unterseite eines Pfropfens aus einem gehärteten dentalen Kompositmaterial (Tetric Evo-Ceram, Fa. Ivoclar Vivadent AG) mit dem Zement benetzt und möglichst mittig auf die Dentinoberfläche platziert. Anschließend wurde der Zahn mit dem Komposit-Pfropfen nach oben in einer Ultradent-Einspannvorrichtung so eingespannt, dass der Fixierungsdorn mittig auf dem Tetric Evo-Ceram-Pfropfen aufsaß. Danach wurde der Überschuss an Befestigungszement sofort sorgfältig entfernt und die Ultradent-Applikationsvorrichtung mit dem eingespannten Zahn für 15 min bei 37 °C im Trockenschrank gelagert. Danach wurden die Pfropfen entlastet, für 24 h in Wasser bei 37 °C aufbewahrt und dann für 24 h im Trockenschrank bei 37 °C gelagert. Zur Messung der Scherhaftung wurden die Pfropfen mittels einer Zwick-Prüfmaschine nach der Ultradent-Methode (EN ISO 29022, Jahr 2013) bei 23 °C abgeschert, wobei sich die Scherhaftfestigkeit als Quotient der Bruchkraft und der Haftfläche ergab.

Mit dem erfindungsgemäßen Zement bestehend aus Basispaste 1 und Katalysatorpaste 1 wurde ein Anfangswert der Dentinhaftung von 12,0 MPa bestimmt. Nach 2 Wochen Lagerung der Pasten bei Raumtemperatur betrug der Wert 10,3 MPa. Demgegenüber ergab der Zement mit dem unbehandelten Füllstoff bestehend aus Basis-paste 2 und Katalysatorpaste 2 einen Anfangswert von nur 1,9 MPa, der nach 2 Wochen Lagerung bei Raumtemperatur auf einen Wert von 0 MPa abfiel.

**Tabelle 1: Zusammensetzung der Basispasten (Angaben in Gew.-%)**

| **Bestandteil** | **Basispaste 1** | **Basispaste 2*)** |
|---|---|---|
| TEGDMA | 8,54 | 8,62 |
| Polyethylenglycol-400-dimethacrylat | 2,51 | 2,53 |
| UDMA | 12,50 | 12,62 |
| N,N-Diethyl-3,5-di-tert.butyl-anilin | 1,498 | 0,600 |
| Campherchinon | 0,075 | 0,076 |
| 4-(Dimethylamino)benzoesäureethylester | 0,151 | 0,152 |
| Benzyltributylammoniumchlorid ¹ | 0,201 | 0,203 |
| Farbpigment (Lumilux LZ Flu Blau)⁶ | 0,00074 | 0,00074 |
| Inhibitor² | 0,00077 | 0,00077 |
| Wasser (deionisiert) | 0,6442 | 0,6501 |
| FAS-Füller (G018-056 7,0 µm, 5% silan.)³ | 69,35 | 69,98 |
| FAS-Füller (G018-056 1,0 µm, 5% silan.)⁴ | 3,05 | 3,08 |
| HDK 2000⁵ | 1,48 | 1,49 |

| | | |
|---|---|---|
| *) Vergleichsbeispiel ¹ Phasentransferkatalysator ² TEMPO: 2,2,6,6-Tetramethylpiperidinyloxyl, CAS-Nummer 2564-83-2 ³ 24 Gew.-% Al₂O₃, 23 Gew.-% SiO₂, 16,5 Gew.-% CaO, 16 Gew.-% CaF₂, 11,5 Gew.-% BaO, 8 Gew.-% P₂O₅, 2 Gew.-% Na₂O, 5% Silan; gewichtsmittlerer Partikeldurchmesser 7 µm (Schott AG, Mainz) ⁴ 24 Gew.-% Al₂O₃, 23 Gew.-% SiO₂, 16,5 Gew.-% CaO, 16 Gew.-% CaF₂, 11,5 Gew.-% BaO, 8 Gew.-% P₂O₅, 2 Gew.-% Na₂O, 5% Silan; gewichtsmittlerer Partikeldurchmesser 1 µm (Schott AG, Mainz) ⁵ pyrogene Kieselsäure; Trimethylsiloxy-Oberflächenmodifizierung; BET-Oberfläche (DIN ISO 9277 DIN 66132) unsilanisiert: ca. 200 m²/g; Dichte (SiO₂; DIN 51757): 2,2 g/cm³; Restsilanol-Gehalt (relativer Silanol-Gehalt bezogen auf unsilanisierte Kieselsäure mit ca. 2 SiOH/nm²): 25 % (Wacker Chemie AG) ⁶ 2,5-Dihydroxyterephthalsäurediethylester (Riedel-de Haën AG) | | |

**Tabelle 2: Zusammensetzung der Katalysatorpasten (Angaben in Gew.-%)**

| **Bestandteil** | **Katalysatorpaste 1** | **Katalysatorpaste 2*)** |
|---|---|---|
| MDP | 3,41 | 3,41 |
| TEGDMA | 10,72 | 10,72 |
| Polyethylenglycol-400-dimethacrylat | 2,38 | 2,38 |
| UDMA | 14,68 | 14,68 |
| Dibenzoylperoxid (50 %) | 0,970 | 0,970 |
| BHT | 0,031 | 0,031 |
| Wasser (deionisiert) | 0,831 | 0,831 |
| Glasfüller GM 27884 1,0 µm, 5% silan.¹ | 63,43 | 63,43 |
| HDK 2000 | 3,54 | 3,54 |

| | | |
|---|---|---|
| *) Vergleichsbeispiel ¹ s. Beispiel 2 | | |

### Beispiel 4

### Untersuchung der Transparenz des Komposits aus Beispiel 3

Von dem Komposit 1 bestehend aus Katalysatorpaste 1 und Basispaste 1 wurde nach vollständiger Härtung die Transparenz mittels eines Spektrophotometers (Konika-Minolta Spektrophotometer CM-5) an 1 mm dicken, auf Hochglanz polierten Prüfkörpern in Transmission gemessen. Die Transparenz betrug 19,2%. Der Transparenzwert des Kompositzements 1 ist deutlich höher als der klassischer Glasionomerzemente, wie z.B. von Vivaglass CEM PL (Ivoclar Vivadent AG) mit einem Transparenzwert von 6,7 %.

### Beispiel 5

### Untersuchung der Lagerstabilität von Kompositen auf der Basis eines röntgenopaken Glasfüllstoffs (mit und ohne Säurebehandlung)

Ein experimenteller röntgenopaker Glasfüllstoff (mittlere Partikelgröße 3 µm; Zusammensetzung (Gew.-%): Al₂O₃: 6; B₂O₃: 5; Na₂O: 8; CaO, BaO, K₂O: je 2-3; CaF₂, MgO: je 1; und SiO₂: 70) wurde auf die in Beispiel 1 beschriebene Weise mit Säure behandelt und silanisiert. Für Vergleichszwecke wurde ein Teil des Füllstoffs ohne vorherige Säurebehandlung silanisiert. In der sauren Behandlungslösung wurden mit XRF-Analyse vor allem Al-, Ba-, Ca-, Na- und K-Ionen nachgewiesen. Sowohl mit dem säurebehandelten Füllstoff als auch mit dem unbehandelten Füllstoff wurden Kompositpasten mit der folgenden Zusammensetzung hergestellt (Gew.-%): Füllstoff: 65,00, MDP: 3,29, TEGDMA: 8,53, NK-Ester 9G: 1,90, V-392: 11,68, BHT: 0,03, deionisiertes Wasser: 5,58 und pyrogene Kieselsäure HDK 2000 (Wacker Chemie AG): 4,00.

Die Pasten mit dem säurebehandelten Füllstoff und mit dem unbehandelten Füllstoff wurden bei Raumtemperatur gelagert und in Abstand von einigen Wochen der Gehalt an MDP mittels HPLC analog Beispiel 2 bestimmt. Die Ergebnisse sind in Fig. 2 dargestellt. Die in Fig. 2 gezeigten Ergebnisse belegen eine deutlich verbesserte Lagerstabilität der Paste auf Basis des mit Säure behandelten Füllstoffs. In der Paste mit dem unbehandelten Füllstoff wurde eine deutliche Annahme des MDP-Gehaltes nach 2 Wochen beobachtet, während in der Paste mit dem säurebehandelten Füllstoff auch nach 8 Wochen keine Abnahme des MDP-Gehaltes festgestellt wurde. Die Ergebnisse zeigen, dass die Säurebehandlung des Füllstoffs die Lagerstabilität deutlich verbessert.

## Patentansprüche

1. Radikalisch polymerisierbare Zusammensetzung, die mindestens ein radikalisch polymerisierbares Monomer ohne Säuregruppe, mindestens ein säuregruppenhaltiges radikalisch polymerisierbares Monomer, mindestens einen Fluoroaluminiumsilikatglasfüllstoff und/oder röntgenopaken Glasfüllstoff und mindestens einen Initiator für die radikalische Polymerisation enthält, **dadurch gekennzeichnet, dass** der Fluoroaluminiumsilikatglasfüllstoffs und/oder Glasfüllstoff mit Säure gewaschener ist.

2. Zusammensetzung nach Anspruch 1, die einen röntgenopaken Glasfüllstoff mit der Zusammensetzung (Gew.-%):
SiO₂: 20-80; B₂O₃: 2-15, BaO oder SrO: 0-40; Al₂O₃: 2-20; CaO und/oder MgO: 0-20; Na₂O, K₂O, Cs₂O: je 0-10; WO₃: 0-20; ZnO: 0-20; La₂O₃: 0-10; ZrO₂: 0-15; P₂O₅: 0-30; Ta₂O₅, Nb₂O₅ oder Yb₂O₃: 0-5; und CaF₂ und/oder SrF₂ 0-10; oder vorzugsweise SiO₂: 50-75; B₂O₃: 2-15; BaO oder SrO: 2-35; Al₂O₃: 2-15; CaO und/oder MgO: 0-10; und Na₂O: 0-10; und/oder einen Fluoroaluminiumsilikatglasfüllstoff mit der Zusammensetzung (Gew.-%): SiO₂: 20-35; Al₂O₃: 15-35; BaO oder SrO: 10-25; CaO: 0-20; ZnO: 0-15; P₂O₅: 5-20; Na₂O, K₂O, Cs₂O: je 0-10; und CaF₂: 0,5-20 Gew.-%; oder vorzugsweise SiO₂: 20-30; Al₂O₃: 20-30; BaO oder SrO: 10-25; CaO: 5-20; P₂O₅: 5-20; Na₂O: 0-10; und CaF₂: 5-20 Gew.-% enthält, wobei sich alle Angaben auf die Gesamtmasse des Glases beziehen und wobei alle Komponenten mit Ausnahme von Fluor als Oxide berechnet werden.

3. Zusammensetzung nach Anspruch 1 oder 2, bei der der Fluoroaluminiumsilikatglasfüllstoff oder Glasfüllstoff mit Salzsäure, Salpetersäure, Ameisensäure und/oder Essigsäure gewaschen ist.

4. Zusammensetzung nach einem der Ansprüche 1 bis 3, die
a) 10 bis 80 Gew.-%, bevorzugt 20 bis 75 Gew.-% und besonders bevorzugt 30 bis 70 Gew.-% mindestens eines Fluoroaluminiumsilikatglasfüllstoffs und/oder Glasfüllstoffs, der mit Säure gewaschen ist,
b) ggf. 0,1 bis 25 Gew.-%, bevorzugt 1 bis 20 Gew.-% und besonders bevorzugt 2 bis 15 Gew.-% eines oder mehrerer weiterer Füllstoffe,
c) 1 bis 15 Gew.-%, bevorzugt 2 bis 12 Gew.-% und besonders bevorzugt 3 bis 10 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,1 bis 8 Gew.-%, bevorzugt 0,5 bis 6 Gew.-% und besonders bevorzugt 1 bis 5 Gew.-% eines Initiators für die radikalische Polymerisation,
h) 0 bis 20 Gew.-%, bevorzugt 0,2 bis 10 Gew.-% und besonders bevorzugt 1 bis 7 Gew.-% an Wasser und
i) 0,01 bis 5 Gew.-%, bevorzugt 0,1 bis 3 Gew.-% und besonders bevorzugt 0,1 bis 2 Gew.-% eines oder mehrerer Additive, jeweils bezogen auf die Gesamtmasse der Zusammensetzung.

5. Zusammensetzung nach einem der Ansprüche 1 bis 4, die als radikalisch polymerisierbares Monomer ohne Säuregruppe mindestens ein polyfunktionelles Monomer (d) enthält, das aus Bisphenol-A-dimethacrylat, Bis-GMA (ein Additionsprodukt aus Methacrylsäure und Bisphenol-A-diglycidylether), ethoxy- oder propoxyliertes Bisphenol-A-dimethacrylaten, wie Bisphenol-A-dimethacrylat mit 3 Ethoxygruppen oder 2,2-Bis[4-(2-methacryloyloxypropoxy)-phenyl]propan, UDMA (ein Additionsprodukt aus 2-Hydroxyethylmethacrylat und 2,2,4-Trimethylhexamethylen-1,6-diisocyanat), Tetramethylxylylendiurethanethylenglycoldi(meth)acrylat bzw. Tetramethylxylylendiurethan-2-methylethylenglykoldiurethandi(meth)acrylat (V380), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.0^{2,6}]decan (DCP), Polyethylenglycol- oder Polypropylenglycoldimethacrylaten, wie Polyethylenglycol-200-dimethacrylat (PEG-200-DMA) oder Polyethylenglycol-400-dimethacrylat (PEG-400-DMA), 1,12-Dodecandioldimethacrylat, Urethanen aus 2-(Hydroxymethyl)acrylsäure und Diisocyanten, wie 2,2,4-Trimethylhexamethylendiisocyanat oder Isophorondiisocyanat, Pyrrolidonen, wie z.B. 1,6-Bis(3-vinyl-2-pyrrolidonyl)-hexan, Bisacrylamiden, wie Methylen- oder Ethylenbisacrylamid, Bis(meth)acrylamiden, wie N,N'-Diethyl-1,3-bis(acrylamido)-propan, 1,3-Bis(methacrylamido)propan, 1,4-Bis(acrylamido)-butan oder 1,4-Bis(acryloyl)-piperazin, und Mischungen davon ausgewählt ist.

6. Zusammensetzung nach einem der Ansprüche 1 bis 5, die als säuregruppenhaltiges Monomer (c) mindestens ein Monomer mit einem pKa-Wert von 0,5 bis 4,0, besonders bevorzugt 1,0 bis 3,5 und ganz besonders bevorzugt 1,5 bis 2,5 bei Raumtemperatur enthält.

7. Zusammensetzung nach einem der Ansprüche 1 bis 6, die als säuregruppenhaltiges Monomer (c) mindestens ein Monomer enthält, das aus Monomeren, die eine Phosphorsäureestergruppe oder Phosphonsäuregruppe enthalten, vorzugsweise 2-Methacryloyloxyethylphenyl-hydrogenphosphat, 10-Methacryloyloxydecyl-dihydrogenphosphat (MDP) Glycerindimethacrylatdihydrogenphosphat, Dipentaerythritpentamethacryloyloxyphosphat, 4-Vinylbenzylphosphonsäure, 2-[4-(Dihydroxyphosphoryl)-2-oxa-butyl]-acrylsäure und/oder 2-[4-(Dihydroxyphosphoryl)-2-oxa-butyl]-acrylsäure-2,4,6-trimethylphenylester, und/oder 4-(Meth)acryloyloxyethyltrimellitsäureanhydrid, 10-Methacryloyloxydecylmalonsäure, N-(2-Hydroxy-3-methacryloyloxypropyl)-N-phenylglycin und/oder 4-Vinylbenzoesäure ausgewählt ist.

8. Zusammensetzung nach einem der Ansprüche 4 bis 7, die als oligomere Carbonsäure (e) Polyacrylsäure mit einer zahlenmittleren Molmasse kleiner 7200 g/mol, bevorzugt kleiner 7000 g/mol und besonders bevorzugt kleiner 6800 g/mol enthält.

9. Zusammensetzung nach einem der Ansprüche 1 bis 8, die als radikalisch polymerisierbares Monomer ohne Säuregruppe mindestens ein monofunktionelles Monomer (f) enthält, das aus Benzyl-, Tetrahydrofurfuryl(meth)acrylat, Isobornyl(meth)acrylat, p-Cumyl-phenoxyethylenglycolmethacrylat (CMP-1E) und 2-([1,1'-Biphenyl]-2-oxy)ethylmethacrylat (MA-836), Tricyclodecan-methyl(meth)acrylat, 2-(2-Biphenyloxy)ethyl(meth)acrylat, 2-Hydroxyethyl-(methacrylat), Hydroxyethylpropyl(methacrylat), 2-Acetoxyethylmethacrylat und Mischungen davon ausgewählt ist.

10. Zusammensetzung nach einem der Ansprüche 4 bis 9, die eine Katalysatorpaste und eine Basis-Paste umfasst, wobei die Katalysatorpaste
a) 10 bis 80 Gew.-%, bevorzugt 20 bis 75 Gew.-% und besonders bevorzugt 30 bis 70 Gew.-% mindestens eines säurebehandelten FAS- und/oder Glasfüllstoffs,
b) 0,1 bis 25 Gew.-%, bevorzugt 1 bis 20 Gew.-% und besonders bevorzugt 2 bis 15 Gew.-% eines oder mehrerer weiterer Füllstoffe,
c) 2 bis 30 Gew.-%, bevorzugt 4 bis 24 Gew.-% und besonders bevorzugt 6 bis 20 Gew.-% mindestens eines säuregruppenhaltigen Monomers,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
e) 0 bis 10 Gew.-%, bevorzugt 0 bis 8 Gew.-% und besonders bevorzugt 1 bis 5 Gew.-% einer oder mehrerer oligomerer Carbonsäuren,
f) 1 bis 20 Gew.-%, bevorzugt 2 bis 15 Gew.-% und besonders bevorzugt 3 bis 10 Gew.-% eines oder mehrerer monofunktioneller Monomere,
g) 0,01 bis 16 Gew.-%, bevorzugt 0,2 bis 12 Gew.-% und besonders bevorzugt 0,5 bis 10 Gew.-% mindestens eines Peroxids und/oder Hydroperoxids ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0,0051 bis 2 Gew.-% eines oder mehrerer Additive enthält, jeweils bezogen auf die Gesamtmasse der Katalysatorpaste,
und wobei die Basis-Paste
a) 10 bis 80 Gew.-%, bevorzugt 20 bis 75 Gew.-% und besonders bevorzugt 30 bis 70 Gew.-% mindestens eines säurebehandelten FAS- und/oder Glasfüllstoffs,
b) 0,1 bis 25 Gew.-%. bevorzugt 1 bis 20 Gew.-% und besonders bevorzugt 2 bis 15 Gew.-% eines oder mehrerer weiterer Füllstoffe,
d) 5 bis 40 Gew.-%, bevorzugt 8 bis 30 Gew.-% besonders bevorzugt 10 bis 25 Gew.-% mindestens eines polyfunktionellen Monomers ohne Säuregruppen,
f) 1 bis 20 Gew.-%, bevorzugt 2 bis 15 Gew.-% und besonders bevorzugt 3 bis 10 Gew.-% eines oder mehrerer monofunktioneller Monomere ohne Säuregruppen,
g) 0,01 bis 16 Gew.-%, bevorzugt 0,3 bis 12 Gew.-% und besonders bevorzugt 1 bis 10 Gew.-% mindestens eines geeigneten Reduktionsmittels und ggf. mindestens eines Photoinitiators,
h) 0 bis 20 Gew.-%, bevorzugt 0,2 bis 10 Gew.-% und besonders bevorzugt 1 bis 7 Gew.-% Wasser und
i) 0,001 bis 5 Gew.-%, bevorzugt 0,002 bis 3 Gew.-% und besonders bevorzugt 0.0051 bis 2 Gew.-% eines oder mehrerer Additive enthält, jeweils bezogen auf die Gesamtmasse der Basis-Paste.

11. Zusammensetzung nach einem der Ansprüche 1 bis 10 zur therapeutischen Anwendung als Dentalwerkstoff, vorzugsweise als dentaler Zement, Beschichtungs- oder Verblendmaterial, Füllungskomposit oder Befestigungszement.

12. Nicht-therapeutische Verwendung einer Zusammensetzung gemäß einem der Ansprüche 1 bis 10 zur Herstellung oder Reparatur von Dentalrestaurationen, insbesondere von Inlays, Onlays, Kronen oder Brücken.

13. Verfahren zur Behandlung eines Fluoroaluminiumsilikatglasfüllstoffs oder Glasfüllstoffs mit Säure, bei dem man
(i) den Fluoroaluminiumsilikatglasfüllstoff oder Glasfüllstoff in einer wässrigen Lösung einer organischen und/oder anorganischen Säure, vorzugsweise Salzsäure, Salpetersäure, Ameisensäure und/oder Essigsäure, dispergiert, wobei die Säurelösung vorzugsweise eine Säurekonzentration von 0,1 bis 5 mol/l, besonders bevorzugt von 0,5 bis 3 mol/I aufweist,
(ii) man die Dispersion rührt, vorzugsweise für 0,5 bis 24 h, besonders bevorzugt für 1 bis 5 h,
(iii) man dann den Füllstoff abtrennt und mit deionisiertem Wasser wäscht und
(iv) man dann den Füllstoff abtrennt und trocknet.

14. Verfahren nach Anspruch 13, bei dem man in Schritt (iii) der Füllstoff in deionisiertem Wasser dispergiert und man dann die Dispersion für 1 bis 60 Minuten, vorzugsweise für 2 bis 20 Minuten rührt und man diesen Vorgang vorzugsweise 1 bis 5 mal, besonders bevorzugt 3 mal wiederholt.

15. Verwendung eines Fluoroaluminiumsilikatglasfüllstoffs oder Glasfüllstoffs, der gemäß Anspruch 13 oder 14 herstellbar ist, zur Stabilisierung dentaler, radikalisch polymerisierbarer Zusammensetzungen.
